Europäisches Patentamt

European Patent Office

Office européen des brevets

(19)

(11) Publication number: **0 007 229**
**B1**

(12) **EUROPEAN PATENT SPECIFICATION**

(45) Date of publication of patent specification: **27.06.84**

(51) Int. Cl.³: **G 01 N 33/58**

(21) Application number: **79301367.3**

(22) Date of filing: **11.07.79**

(54) Assay involving labelled substances employing a re-usable immobilised binder.

(30) Priority: **12.07.78 US 923711**

(43) Date of publication of application:
**23.01.80 Bulletin 80/2**

(45) Publication of the grant of the patent:
**27.06.84 Bulletin 84/26**

(84) Designated Contracting States:
**BE DE GB IT NL SE**

(56) References cited:
**DE - A - 2 603 004**
**DE - A - 2 607 149**
**DE - A - 2 752 352**
**DE - C - 413 690**
**US - A - 3 896 217**
**US - A - 4 009 005**
**US - A - 4 059 685**
**US - A - 4 108 976**

(73) Proprietor: **Becton, Dickinson and Company**
**Mack Centre Drive**
**Paramus New Jersey 07652 (US)**

(72) Inventor: **Reese, Max G.**
**977 E. 5600 South**
**Salt Lake City, Utah (US)**

(74) Representative: **Ruffles, Graham Keith et al,**
**MARKS & CLERK 57-60 Lincoln's Inn Fields**
**London WC2A 3LS (GB)**

Courier Press, Leamington Spa, England.

# Description

This invention relates to an assay of the kind employing a re-usable immobilized binder.

United States Patent 3,896,217 discloses an assay in which a binder covalently bound to a substrate is regenerated by flowing an eluting solution therethrough. By this system, the binder may be repeatedly re-used.

An automated assay is described in U.S. Patent No. 4,009,005 which sets forth in more detail the various aspects of equipment for an assay employing a re-usable binder.

US Patent No. 4059685 describes a reusable immobilised immunoadsorbent for use in such automated assays: antibody is covalently linked to a barrier coating of polymer itself bound by silane linkages to the outer porous coating of superficially porous refractory particles comprising an impervious core and layers of microparticles making up the porous coating. Principally this patent discloses the use of dextran solution to form the polymer barrier coating, but cellulose is also mentioned.

The systems of the above U.S.Patents are principally of the flow-through type. With such systems plugging can be a problem, unlike the case with the prior art systems which employ a single use binder. If the flow passages of a flow-through system become plugged up then the use and re-use of the binder is adversely affected and there is consequential interference with subsequential assays. Adverse affects of the physical plugging include initially a decrease in the speed of the assay stemming from the decrease in the flow rate. Ultimately the flow passages become blocked to the point where flow is prevented.

One source of pluging is the proteins, other than the substance to be analyzed, which may be present in the sample. If undiluted serum is assayed, the relatively large proteins tend to plug up the immobilized binder. The serum may be diluted to reduce the concentration of proteins for better flow through the immobiilized binder; however, such dilution reduces the concentration of the substance to be assayed in the serum. Where the concentration of substance is relatively low, as is usually the case for example with digoxin or $T_3$ (thiodothyronine), then diluting the serum reduces the concentration even more and presents problems in accurately and reliably assaying for the relatively small amounts of substance present in the diluted serum. Thus, dilution to avoid plugging problem leads to problems of accurate assay; the sensitivity becomes less than is desired.

One aspect of the present invention concerns an improvement in an assay wherein a substance to be assayed and a labelled form of the substance are caused to flow through a chamber having a binder specific for the substance supported on a solid substrate and wherein the binder is regenerated for re-use in a further assay by releasing unlabelled and labelled substance from the binder. Another aspect concerns a chamber for use in such an assay.

Both aspects of the invention are characterised by the use of fibrous cotton as the solid substrate on which the binder is supported. Thus, the solid substrate consists of fibrous cotton, unlike the idea in U.S. Patent 4059685 where the polymer which may be cellulose is coated onto a refractory core.

We find that a cotton substrate usually does not lead to plugging when serum or plasma samples are passed therethrough. In addition, a cotton substrate does not compress when tightly packed, even if the flow rate through the chamber is high. In contrast, a conventional immobilized binder employing beads of an agarose gel as support material compacts as the flow rate increases. It appears that the known beads, which are usually of Sephadex or Sepharose (both registered trade marks of Pharmacia A.B. of Uppsala, Sweden), are subject to hydrostatic collapse an eventually block the flow of liquid through the chamber.

Moreover, a typical cotton substrate has good flow properties, is easily handled, can be easily derivatized for covalent linking of binder thereto, has low non-specific binding of proteins and steroids, and is stable.

The cotton employed as the support is fibrous cotton and can be woven e.g. a woven cotton gauze, or non-woven e.g. balls of cotton, with the cotton preferably being a woven gauze. A loosely woven material permits the use of a greater amount of material in a given volume. The cotton is most preferably an absorbent cotton and in particular mercerized absorbent cotton; such a cotton has greater binder capacity. Thus if the cotton has not been mercerized it is preferably treated with sodium hydroxide or the like reagent before use.

A binder may be conveniently supported on the cotton substrate employed in the present invention by effecting activation of the substrate with cyanogen bromide using any of the procedures known generally in the art, followed by conjugation, normally through covalent linking, of the binder to the cotton. In brief, a typical procedure involves contacting the cotton substrate with cyanogen bromide at a basic pH, generally in the order of from pH 10.0 to pH 12.0, followed by washing and air-drying. The binder or receptor to be used in the assay may then be covalently bonded to the thus activated cotton substrate by any of the procedures known in the art.

A general procedure for effecting activation of dextran, cellulose or like material using cyanogen bromide and subsequent covalent linking of a binder is described in U.S. Patent No. 4,059,685; such a procedure may be employed for covalently linking a binder to a cotton substrate employed in the present invention.

Although activation by the use of cyanogen bromide is preferred, other procedures for covalently linking a binder to a cotton substrate may be employed. Thus, using for example a technique described in U.S. Patent No. 4,108,976, published on 22 August 1978, a cotton substrate may be treated with a halogenated acid such as bromoacetic acid to form a carboxyl derivative, which may then be treated with thionyl chloride to form the acid chloride which is then further treated with sodium azide to form an acylazide derivative to which a binder can be conjugated.

The cotton substrate having a binder covalently linked thereto can be employed in an assay by placing such substrate in a chamber of the type described in U.S. Patent No. 4,059,685 and used in an automated assay of the type described in U.S. Patent No. 4,009,005. The reader is referred to these two patents for details. Put briefly, in accordance with such an assay, the substance to be assayed and a labelled form of such substance are caused to flow through a chamber containing a binder specific for the substance covalently attached to the cotton substrate. A portion of both the labelled and unlabelled substance become bound to the binder in the chamber. The remainder of the labelled and unlabelled substance passes through the chamber, preferably to a detector where it is quantitatively determined. Subsequently, an eluting solution is flowed through the chamber to free both the labelled and unlabelled substance bound to the immobilized binder. The labelled and unlabelled substance freed from such binder by the eluting solution flows to a detector where it also is usually quantitatively determined. Following elution to free the substance, the binder covalently linked to the cotton substrate is rinsed and ready for re-use in the next and subsequent cycles. From the or both quantitative determinations of each assay, the amount of the substance in each original sample can be determined by reference to a standard curve.

In such a process in accordance with the present invention, it has been found that samples can be caused to pass through such a chamber without plugging occurring, and without the necessity of effecting high dilution of such samples. Thus, there is no need for a significant reduction in the concentration of the substance to be assayed, and the sensitivity of the assay can remain high. Thus, for example, it has been found that samples of high serum content; e.g. up to 100 percent serum, can be employed without plugging.

The present invention is applicable to assays for a wide variety of substances for which an appropriate binder can be found. Suitable substances include antigens, i.e. substances which when introduced into the blood stream of a vertebrate result in the formation of antibodies specific for the antigen; haptens, i.e. substances which when bound to an antigenic carrier and introduced into the blood stream of a vertebrate produce antibodies specific for the hapten (haptens are sometimes interchangeably referred to as antigens); or other substances which have naturally occurring binders which can be isolated in a form specific for the substance, such as, for example, serum proteins (thyroxine binding globulin, which is a binder for thyroxine, and triiodothyronine), binders extracted from various animal organs, serums, milk binders and the like. It is also to be understood that in some cases, the substance to be assayed is an antibody, in which the binder would be an antigen.

As representative classes of substances which can be assayed using an assay in accordance with the present invention, there may be mentioned: proteins and other peptides, nucleotides, nucleosides saccharides, steroids and derivatives of such compounds. Representative examples of such compounds are ACTH oxytocin, luteniizing hormone, insulin, proinsulin, Bence-Jones protein, chorionic gonadotropin, pituitary gonadotropin, growth hormone, rennin, thyroxine-binding globulin, bradykinin, antiotensin, follicle-stimulating horbome; cyclic AMP; cholyglycine (glycocholic acid), cyclic GMP, estrogens, gestrogens, androgens, adrenocotical hormones, bile acids, cardiotonic glycosides, aglycones and saponins. As further examples of substances there may be mentioned: thyroxine, triiodothyronine, testosterone, androsterone, equilienin, estrone, estriol, progesterone, pregenolone, 17-hydroxy-dioxycortiocosterone (compound S), deoxycorticoserone, cortisone, corticosterone, cortisol, aldosterone, digoxin, digitoxini, vitamins, such as folic acid, the B vitamin group, the D vitamins and miscellaneous substances such as antigens for Viral Hepatitis A and B, Rubelle, Herpes Simplex $\alpha$-fetoprotein, etc.

The labelled substance employed in the assay can be the substance or an appropriate analog thereof, provided that the substance to be assayed and labelled substance are both specifically bound by the binder employed in the assay. The label may be any one of a wide variety of labels or tags, including radioisotopes, enzymes, fluorescent materials, etc. A radioisotope, such as a radioisotope of iodine, tritium, cobalt or other element is preferred.

The present invention is illustrated by the following non-limiting example.

Example: Digoxin assay

In themselves the techniques employed in this Example are well-known and a detailed description is unnecessary. The procedure for the Example is as follows:

Mercerization of cotton
1. Immerse cotton gauze in 4N NaOH, flush container with nitrogen, seal and store 24 hours at 4°C.
2. Wash thoroughly the next day with distilled water until the washes are neutral pH. Wash twice in phosphate buffer pH 7.5 and once again with distilled water. Pat dry and store in air tight container.

II Activation of cotton

1. Dissolve 20 grams CNBr in 200 ml distilled water.

2. In a separate vessel place the cotton (approximately 1.5 g) in 400 ml phosphate buffer 0.5 M pH 11.0 and stir. Maintain the temperature at 4° to 10°C.

3. Add the 20 grams dissolved CNBr to the stirring cotton slowly. Total time of reaction approximately 10 minutes.

4. Wash the cotton with pre-chilled distilled water.

5. Wash the cotton with increasing concentrations of acetone in water to reach 100% acetone. The cotton is now ready to store dry or for use immediately for conjugating of antisera.

III Conjugation

1. One ml of rabbit or sheep anti-digoxin antiserum with one ml of 0.1M sodium bicarbonate is dialyzed against distilled water at room temperature for 1 hour and then overnight at 4°C against 0.1M sidium bicarbonate.

2. Approximately 200 mg of activated cotton is used to conjugate 50 $\mu$l of the above dialyzed antiserum diluted in 1.5 ml of 0.1M sodium bicarbonate at pH 9.5. The conjugation vessel is sealed and then agitated at 4°C for three days.

3. The conjugate is washed in distilled water and phosphate buffer 0.05M pH 7.5. Store in the phosphate buffer 0.05M pH 7.5 at 4°C.

IV Chamber preparation

1. A teflon chamber with an inside diameter of 0.149 inches (about 3.8 mm) is filled with the conjugate (approximately 50 mg).

2. The end pieces of the chamber are pressed into place and the chamber is ready for use.

V. Digoxin assay: Protocol

A. Reagents and materials

1. Digoxin antibody chamber: containing antibody covalently attached to cotton.

2. Digoxin standards: containing digoxin in stabilized 100% human serum made up to the following concentrations:—

0
0.5 ng/ml
1.0 ng/ml
2.0 ng/ml
4.0 ng/ml
8.0 ng/ml

3. Adsorption buffer: phosphate buffer 0.05M, pH 7.5, and 0.15M sodium chloride.

4. Elution buffer: phosphate buffer 0.05M, pH 8.25, diluted with equal volumes of methanol.

5. $^{125}$I digoxin tracer.

B Performance

Automated apparatus: as described in U.S. Patent No. 4,009,005, available from Becton, Dickinson Immunodiagnostics, Automated Immunochemistry Systems and identified by the registered trade mark ARIA II.

C. Calculations

Performed by self-container computer of the ARIA II apparatus. Each unknown sample is compared with calculated standard curve previously run, using a weighted regression analysis. Results are printed out on a permanent record, including for each unknown sample the concentration of digoxin in ng/ml.

VI Digoxin assay: Conclusions

The present assay is particularly advantageous in that an assay including regeneration and re-use of the binder can be effected without plugging of the chamber including immobilized binder, and without the necessity of effecting high dilution of the serum samples and/or taking preliminary steps to avoid such plugging. By employing a cotton substrate in accordance with the present invention we find plugging problems are eliminated, while retaining the advantages of the supports previously employed in such assays, i.e. good flow properties, low non-specific binding of proteins and steroids, and the like.

Numerous modifications and variations of the present invention are possible. For example using an appropriate binder it is possible to assay for triiodothyronine (T$_3$) or thyroid stimulating hormone (TSH).

**Claims**

1. An assay wherein a substance to be assayed and a labelled form of the substance are caused to flow through a chamber containing a binder which is specific for the substance and is supported on a solid substrate, and wherein the binder is regenerated for re-use in a further assay by releasing unlabelled and labelled substance from the binder, characterized in that the solid substrate is made of fibrous cotton.

2. An assay according to Claim 1, wherein the cotton is an absorbent cotton.

3. An assay according to Claim 2, wherein the absorbent cotton is mercerized absorbent cotton.

4. An assay according to any preceding claim, wherein the cotton is in the form of a woven cotton gauze.

5. An assay according to any preceding claim, wherein the binder is covalently linked to the cotton support.

6. An assay according to any preceding claim, wherein the labelled substance is radiolabelled.

7. An assay according to any preceding claim, wherein the binder is an antibody.

8. An assay according to any preceding claim, wherein the substance to be assayed is in a serum sample.

9. A chamber for use in an assay according to claim 1, the chamber being arranged to receive flow therethrough of substance to be analysed and containing a binder specific to such substance, the binder being supported on a substrate, characterized in that the substrate is made of fibrous cotton.

## Patentansprüche

1. Prüfung, bei der eine zu prüfende Substanz und eine markierte Art der Substanz im Strom durch eine Kammer geleitet werden, die ein Bindemittel enthält, das kennzeichnend für die Substanz ist und auf einem festen Substrat abgestützt ist, und das Bindemittel zur Wiederverwendung in einer weiteren Prüfung regeneriert wird, indem unmarkierte und markierte Substanz vom Bindemittel getrennt werden, dadurch gekennzeichnet, daß, das feste Substrat aus faserförmiger Baumwolle hergestellt ist.

2. Prüfung nach Anspruch 1, dadurch gekennzeichnet, daß die Baumwolle von saugfähiger Watte gebildet ist.

3. Prüfung anch Anspruch 2, dadurch gekennzeichnet, daß die Baumwollwatte merzerisiert ist.

4. Prüfung nach einem der vorangehenden Ansprüche, dadurch gekennzeichnet, daß die Baumwolle die Form einer gewebten Baumwollgaze aufweist.

5. Prüfung nach einem der vorangehenden Ansprüche, dadurch gekennzeichnet, daß das Bindemittel kovalent mit der Baumwollunterstützung verkettet ist.

6. Prüfung nach einem der vorangehenden Ansprüche, dadurch gekennzeichnet, daß die markierte Substanz strahlenmarkiert ist.

7. Prüfung nach einem der vorangehenden Ansprüche, dadurch gekennzeichnet, daß das Bindemittel ein Immunkörper ist.

8. Prüfung nach einem der vorangehenden Ansprüche, dadurch gekennzeichnet, daß sich die zu prüfende Substanz in einer Serumprobe befindet.

9. Kammer zur Verwendung bei einer Prüfung gemäß Anspruch 1, die mit zu prüfender Substanz durchströmbar ist und ein für diese Substanz kennzeichnendes Bindemittel enthält, das auf einem Substrat unterstützt ist, dadurch gekennzeichnet, daß das Substrat aus faserförmiger Baumwolle hergestellt ist.

## Revendications

1. Essai selon lequel une substance à soumettre à essai et une forme marquée de la substance sont amenées à s'écouler par une chambre contenant un liant qui est spécifique pour la substance et qui est supporté par un substrat solide, et selon lequel le liant est régénéré en vue de pouvoir être réutilisé au cours d'un essai subséquent par libération de la substance non marquée et de la substance marquée du liant, l'essai étant caractérisé en ce que le substrat solide est fait de coton fibreux.

2. Essai suivant la revendication 1, caractérisé en ce que le coton est un coton absorbant.

3. Essai suivant la revendication 2, caractérisé en ce que le coton absorbant est du coton absorbant mercerisé.

4. Essai suivant l'une quelconque des revendications précédentes, caractérisé en ce que le coton se présente sous la forme d'une gaze de coton tissée.

5. Essai suivant l'une quelconque des revendications précédentes, caractérisé en ce que le liant est lié de façon covalente au support de coton.

6. Essai suivant l'une quelconque des revendications précédentes, caractérisé en ce que la substance marquée est radio-marquée.

7. Essai suivant l'une quelconque des revendications précédentes, caractérisé en ce que le liant est un anticorps.

8. Essai suivant l'une quelconque des revendications précédentes, caractérisé en ce que la substance à soumettre à essai est un échantillon de sérum.

9. Chambre destinée à être utilisée pour l'exécution d'un essai suivant la revendication 1, la chambre étant prévue pour qu'y ait lieu l'écoulement de la substance à analyser et contenant un liant spécifique pour cette substance, le liant étant supporté par un substrat, la chambre étant caractérisée en ce que le substrat est fait de coton fibreux.